# EUROPÄISCHE PATENTSCHRIFT

(11) **EP 1 151 723 B1**
(45) Veröffentlichungstag und Bekanntmachung des Hinweises auf die Patenterteilung: **17.09.2008**
(21) Anmeldenummer: 01110666.3
(22) Anmeldetag: 02.05.2001
(51) Int. Cl.: A61B 17/28, A61B 18/14

(54) **Chirurgisches Instrument, insbesondere Pinzette oder Zange**
Surgical instrument, particularly a pincette or a forceps
Dispositif chirurgical, notamment du type pince ou du type pincette

(30) Priorität: 04.05.2000 DE 10021724; 29.06.2000 DE 10031773
(43) Veröffentlichungstag der Anmeldung: 07.11.2001
(73) Patentinhaber: Erbe Elektromedizin GmbH, 72072 Tübingen (DE)
(72) Erfinder: Bartel, Volker, 72411 Bodelshausen (DE)
(74) Vertreter: Bohnenberger, Johannes

(56) Entgegenhaltungen:
- DE-C- 19 731 884
- US-A- 4 005 714
- US-A- 5 172 700
- US-A- 5 222 973
- US-A- 5 964 780

## Beschreibung

Die Erfindung betrifft ein chirurgisches Instrument , insbesondere eine Pinzette oder Zange für die minimalinvasive Chirurgie, gemäß dem Oberbegriff von Patentanspruch 1.

Derartige chirurgischen Instrumente werden in der minimalinvasiven Chirurgie beispielsweise zum Greifen von Gewebe, kleinen Blutadern oder dergleichen biologischer Materialien eingesetzt. Ähnlich wie eine Zange oder Pinzette weist ein solches Instrument bewegliche Greifteile auf, die zum Greifen in und entgegen einer Greifrichtung zusammen- und auseinander klappbar sind. Die Instrumente können aufgrund ihrer geringen Abmessungen bevorzugt in den Arbeitskanal Endoskopen eingesetzt werden.

Aus der DE 195 37 320 A1 ist eine Vorrichtung zum Betätigen einer Greifvorrichtung für die minimalinvasive Chirurgie bekannt, bei der die Vorrichtung ähnlich einer Zange zwei bewegbare Greifteile als Zangenhälften umfaßt. Die Greifteile sind jeweils über zwei flexible Verbindungsteile mit einem ersten bzw. zweiten Halteteil verbunden. Zum Auseinander- oder Zusammenklappen der beiden bewegbaren Handhabungsteile werden die beiden Halteteile relativ zueinander verschoben. Als Vorteil dieser Vorrichtung ist angegeben, daß sie einerseits keine verschleißbaren Gelenke aufweist und andererseits aus einem einzigen Materialrohling herstellbar ist. Diese Druckschrift ist im Oberbegriff des Anspruches 1 gewürdigt.

Ein Einsatz der vorgenannten Vorrichtung in der minimalinvasiven Chirurgie zum Koagulieren von biologischem Gewebe, beispielsweise als bipolares Instrument, ist jedoch nicht ohne komplizierte, zusätzliche Maßnahmen möglich.

Aus der DE 40 32 471 C2 ist eine elektrochirurgische Vorrichtung bekannt, die wahlweise zur bipolaren Koagulation oder zur Gewebetrennung verwendbar ist und drei, gegeneinander isolierte und in einem länglichen Instrumentengehäuse montierte Kontaktstangen umfaßt. Zwei der Kontaktstangen dienen mit ihren freien Enden als Koagulationselektroden, die dritte Kontaktstange als Schneidelektrode. Die dritte Kontaktstange ist hierzu auf die beiden Koagulationselektroden zu oder durch diese hindurch bewegbar, so dass zu koagulierendes oder zu schneidendes Gewebe zangenartig zwischen die Elektroden eingeklemmt werden kann.

Ferner offenbart die DE 39 17 328 C2 ein bipolares Koagulationsinstrument mit zwei gegeneinander isolierten und beweglichen Maulteilen, die eine Greifzange bilden. Hierbei ist ein erstes Maulteil feststehend und das zweite Maulteil gegenüber dem ersten Maulteil beweglich.

Die beiden vorgenannten Instrumente bzw. Vorrichtungen sind allerdings konstruktiv recht aufwendig und daher entsprechend teuer in der Herstellung.

Aus der DE 197 31 884 C, der US-A-5 172 700, US-A-5 222 973 und der US-A-4 005 714 sind jeweils chirurgische Instrumente bekannt, bei welchen das Öffnen und Schließen der Greifteile dadurch bewerkstelligt wird, dass diese zu einer sie umfassenden Hülse verschoben werden. Derartige Verschiebung von Teilen bringt aber insbesondere bei minimalinvasiven Eingriffen, bei welchen höchste Präzision gefordert ist, Nachteile mit sich.

Der Erfindung liegt die Aufgabe zu Grunde, ein chirurgisches Instrument nach dem Oberbegriff des Patentanspruches 1 aufzuzeigen, das ein Arbeiten mit gesteigerter Präzision bei einfacher Konstruktion ermöglicht.

Diese Aufgabe wird durch ein chirurgisches Instrument mit den Merkmalen von Patentanspruch 1 gelöst. Bevorzugte Ausführungsformen des Instrumentes ergeben sich aus den abhängigen Patentansprüchen.

Ein wesentlicher Punkt der Erfindung liegt darin, daß das Instrument mindestens zwei Greifteile umfaßt, die zum Greifen ähnlich wie eine Zange in und entgegen einer Greifrichtung zusammen- bzw. auseinanderklappbar sind. Die Greifteile weisen an einem Ende jeweils einen ersten und einen zweiten Schenkel mit jeweils einem Biegebereich auf, die in der Greifrichtung voneinander beabstandet sind. Mindestens die ersten Schenkel sind innerhalb einer Führungshülse angeordnet und in Längsrichtung der Führungshülse relativ zum zweiten Schenkel bewegbar, so daß bei einer Relativbewegung der Schenkel zueinander in deren Längsrichtung das jeweilige Greifteil in Greifrichtung bewegbar ist. Mit anderen Worten biegen sich die Schenkel an ihren Biegebereichen, wenn die beiden Schenkel eines Greifteiles relativ zueinander verschoben werden. **Prinzipiell eignet sich das Instrument auch als Schere, wenn die Greifteile mit Schneiden versehen sind. Beispielsweise kann eine Kante mindestens eines Greifteils als Schneide ausgebildet sein.**

Um bei einer bevorzugten Ausführungsform die Greifteile auseinander zu klappen, wird beispielsweise der erste Schenkel jedes Greifteils in Längsrichtung der Führungshülse zu dem Greifende der Greifteile hin bewegt. Aufgrund des dadurch auftretenden Drehmomentes biegen sich die Schenkel an ihrem Biegebereich derart, daß die Greifteile entgegen der Greifrichtung auseinander klappen. Durch Zurückbewegen der ersten Schenkel klappen die Greifteile wieder aufgrund der Rückstellkraft der Biegebereiche bzw. eine entsprechende gegenläufige Bewegung der Schenkel wieder zusammen, wodurch sich zwischen den Greifteilen befindliches Gewebe sicher fassen läßt. Insgesamt weist das Instrument einen einfachen konstruktiven Aufbau auf, der eine besonders kleine Ausführung ermöglicht.

Die Greifteile selbst bestehen in einer bevorzugten Ausführungsform aus einem leitfähigem Material, insbesondere einem Metall, vorzugsweise einem Edelstahl. Die Greifteile sind vorzugsweise so angeordnet, daß sich die Schenkel nicht berühren und somit elektrisch voneinander isoliert sind. Dadurch eignen sich die Greifteile als Elektroden zum Koagulieren von biologischem Gewebe.

Vorzugsweise ist jedes Greifteil mitsamt seinen Schenkeln einstückig aus einem leitfähigen, insbesondere metallenen Material hergestellt. Hierdurch sind die Greifteile sehr kostengünstig herstellbar; denkbar ist auch eine Ausführung aus leitfähigem Kunststoff oder dergleichen leitfähigen Materialien.

In einer besonders bevorzugten Ausführungsform ist der erste Schenkel jedes Greifteils länger als der zweite Schenkel, so daß die Greifteile direkt über die ersten Schenkel, das heißt ohne Gelenke, Stangen, Wellen oder dergleichen Einrichtungen absolut spielfrei betätigbar sind. Zusätzlich können die ersten Schenkel direkt als Zuleitungen für einen Strom zur HF-Koagulation dienen. Zur Betätigung der Greifteile kann beispielsweise an die ersten Schenkel jeweils eine Stange angeschweißt sein. Dies ermöglicht eine Betätigung in großem Abstand von den Greifteilen.

Eine weitere Vereinfachung bei der Produktion des Instrumentes wird dadurch erzielt, daß in einer bevorzugten Ausführungsform der zweite Schenkel jedes Greifteiles insbesondere mit der Innenwand der Führungshülse fest verbunden, beispielsweise formschlüssig verbunden wird. Eine solche Ausführung ist herstellungstechnisch billiger und benötigt weniger Platz als eine Schraubverbindung.

Zum Zuführen eines hochfrequenten Koagulationsstromes zu einem mit dem Instrument gegriffenen Gewebe sind die Schenkel verschiedener Greifteile voneinander elektrisch isoliert, insbesondere mit einem Isolierschlauch umhüllt. Um Kurzschlüsse sicher zu vermeiden, werden die Schenkel der Greifteile vorzugsweise mittels Isolierschlauch vor dem Einbau in die Führungshülse umhüllt. Auch Produktionstoleranzen, aufgrund derer sich Schenkel verschiedener Greifteile berühren könnten, spielen bei einer Isolierung keine Rolle mehr.

Zur Handhabung des Instrumentes ist die Führungshülse teilweise in ein Ende eines Schlauches oder insbesondere metallenen Rohres eingeführt. Das Rohr kann insbesondere aus einem Edelstahl bestehen, der den in der Chirurgie eingesetzten Edelstahltypen entspricht. Ein Schlauch bzw. Rohr erleichtert die Handhabung des Instrumentes, bietet vor allem einen guten Schutz vor Verschmutzungen und läßt sich für chirurgische Eingriffe leichter reinigen und sterilisieren.

Vorzugsweise ist der Schlauch oder das Rohr an dem der Führungshülse gegenüberliegenden Ende mit einem (steuerbaren) Aktuator oder einem Handhabungsgriff zum Handhaben des Instruments, insbesondere zur Betätigung der Greifteile, d. h. zum Auseinander- oder Zusammenklappen, versehen. Mit dem Handhabungsgriff kann das chirurgische Instrument von einem Operateur einfach und bequem gehandhabt werden.

In einer weiteren Ausgestaltung des Handhabungsgriffes sind die ersten Schenkel der Greifteile mit einer Betätigungseinrichtung verbunden, die derart in dem Handhabungsgriff gelagert ist, daß durch Einführen der Betätigungseinrichtung in den Handhabungsgriff die ersten Schenkel in Längsrichtung der Führungshülse und des Schlauches oder Rohres vom Handhabungsgriff weg bewegt werden, wodurch die Greifteile entgegen der Greifrichtung auseinander klappen. Umgekehrt klappen die Greifteile wieder in Greifrichtung zusammen, wenn die Betätigungseinrichtung aus dem Handhabungsgriff herausgezogen und damit die ersten Schenkel wieder zurückgezogen werden.

Zwischen Betätigungseinrichtung und Handhabungsgriff ist vorzugsweise eine Feder angeordnet, so dass ohne äußere Krafteinwirkung die Betätigungseinrichtung aus dem Handhabungsgriff gedrückt wird und die Greifteile zusammen geklappt sind; dies entspricht sozusagen der Ruhestellung des Instruments. Zum Greifen von Gewebe muß ein Operateur dann lediglich die Betätigungseinrichtung in den Handhabungsgriff drücken, so dass die Greifteile auseinander geklappt werden, und nach Ergreifen des Gewebes die Betätigungseinrichtung wieder loslassen, so dass diese aufgrund der Federkraft in ihre Ausgangsstellung zurückgedrückt wird, die Greifteile zusammenklappen und das Gewebe mit definierter Kraft fest greifen.

Zum Einsatz als bipolares Instrument zur HF-Koagulation ist der erste Schenkel eines ersten Greifteiles mit einem ersten Anschluss und der erste Schenkel eines zweiten Greifteiles mit einem zweiten Anschluss eines HF-Stromgenerators verbindbar. Das erste und das zweite Greifteil entsprechen dann zwei gegenpoligen Elektroden und dienen zur Zuleitung von HF-Strom zu einem mit beiden Greifteilen gefassten Gewebe.

Weitere Vorteile der Erfindung ergeben sich aus der nachfolgenden Beschreibung von Ausführungsbeispielen in Verbindung mit den Zeichnungen. Hierbei zeigen:
- - Fig. 1: ein erstes Ausführungsbeispiel des erfindungsgemäßen chirurgischen Instrumentes teilweise im Längsschnitt;
- - Fig. 2: das erste Ausführungsbeispiel des chirurgischen Instrumentes mit Handhabungsgriff und Betätigungseinrichtung;
- - Fig. 3: das in Fig. 2 dargestellte Ausführungsbeispiel im Längsschnitt entlang der Linie III - III aus Fig. 2;
- - Fig. 4: ein zweites Ausführungsbeispiel des erfindungsgemäßen chirurgischen Instruments mit zusammengeklappten Greifteilen in perspektivischer Ansicht;
- - Fig. 5: eine Seitenansicht des in Fig. 4 dargestellten zweiten Ausführungsbeispiels des chirurgischen Instrumentes;
- - Fig. 6: einen Teilschnitt längs der Linie VI - VI des in Fig. 5 dargestellten Ausführungsbeispiels;
- - Fig. 7: das in Fig. 4 dargestellte Ausführungsbeispiel des Instrumentes mit auseinander geklappten Greifteilen;
- - Fig. 8: eine Seitenansicht des in Fig. 7 dargestellten Instrumentes; und
- Fig. 9: einen Teilschnitt längs der Linie IX - IX des in Fig. 8 dargestellten Instrumentes;

In der folgenden Beschreibung sind gleiche oder gleich wirkende Elemente mit denselben Bezugszeichen versehen.

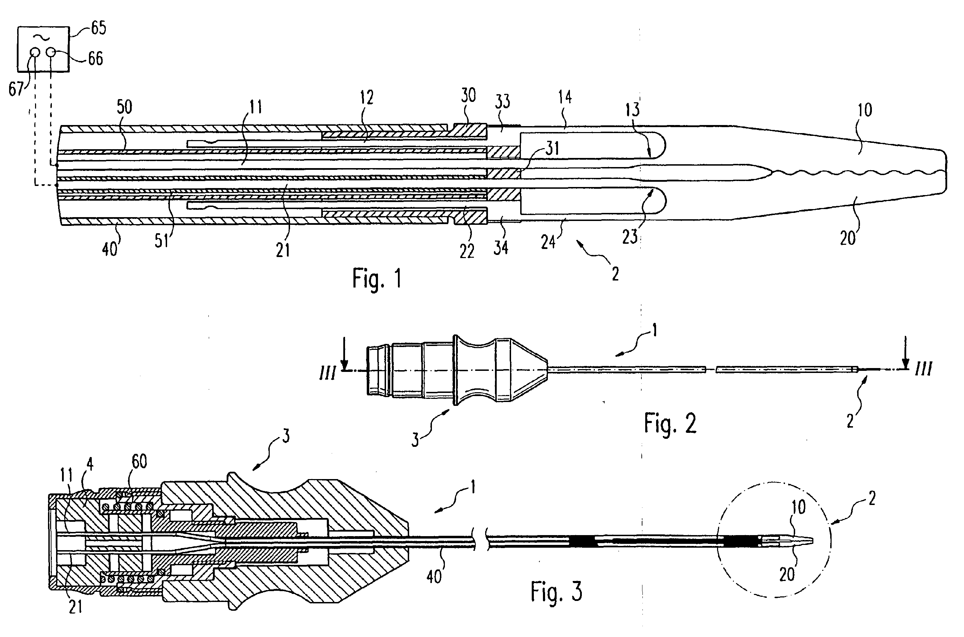

In Fig. 1 ist die Instrumentenspitze 2 eines ersten Ausführungsbeispiels des chirurgischen Instruments gemäß der Erfindung dargestellt. Zwei Greifteile 10 und 20 sind in einer Führungshülse 30 gelagert, die wiederum teilweise in einem Ende eines Rohres 40 steckt. Das Rohr 40 dient einerseits als schützende Außenhülle und andererseits als Führung für die Instrumentenspitze. Die Führungshülse 30 besteht vorzugsweise aus einem isolierenden Material, insbesondere einem Kunststoff. Die Führungshülse 30 führt erste oder innere Schenkel 11 und 21 und zweite oder äußere Schenkel 12 und 22 des ersten bzw. des zweiten Greifteils 10 bzw. 20. Schenkel und Greifteile sind jeweils einstückig aus einem leitfähigen Material, insbesondere einem Edelstahl wie Nitinol (NiTi)hergestellt. Nitinol weist eine große Dehnung von 8% gegenüber Stahl mit einer Dehnung von nur 0,2% auf und eignet sich daher besonders gut für die Schenkel. An ihren den Schenkeln abgewandten Enden sind die beiden Greifteile 10 und 20 ähnlich wie Zangenhälften ausgebildet und passen insbesondere im zusammengeklappten Zustand der Greifteile so zusammen, daß eine Profilierung einer Greiffläche des ersten Greifteils 10 in eine entsprechend komplementäre Profilierung einer Greiffläche des zweiten Greifteils 20 eingreift.

Die außerhalb der Führungshülse 30 und des Metallrohrs 40 befindlichen Abschnitte der ersten Schenkel 11 und 21 sowie zweiten Schenkel 12 und 22 weisen jeweils einen Biegebereich 13 bzw. 23 und 14 bzw. 24 auf. Wenn die Greifteile aus Metall bestehen, sind die flexiblen Bereiche federnd ausgebildet. Die zweiten Schenkel 12 und 22 des ersten Greifteils 10 bzw. zweiten Greifteils 20 sind an der Führungshülse 30 festgemacht, insbesondere festgeklebt oder festgeklemmt und stützen sich dort über Vorsprünge 33, 34 gegenüber einer Bewegung in die Führungshülse 30 hinein ab. Die ersten Schenkel 11 und 21 des ersten Greifteils 10 bzw. zweiten Greifteils 20 sind in der Führungshülse 30 beweglich gelagert und mittels eines isolierenden Abstandselementes 31 (ein Teil der Führungshülse 30) voneinander beabstandet. Zur zusätzlichen Isolierung sind die ersten Schenkel 11 und 21 jeweils von einem isolierenden Schlauch 51 umhüllt. Um beim Zusammenbau des Instrumentes zu verhindern, dass Klebstoff an die beweglichen Teile, insbesondere an die ersten Schenkel 11 und 21 gelangt, ist ein weiterer Schlauch 50 als Schutzhülle vorgesehen. Der Schlauch 50 umhüllt hierzu die beweglichen ersten Schenkel 21 und 22.

An den ersten Schenkel 11 und den zweiten Schenkel 21 ist ein HF-Stromgenerator 65 angeschlossen, der einen hochfrequenten Koagulationsstrom liefern kann. Hierzu ist der erste Schenkel 11 mit einem ersten Anschluß 66 des HF-Stromgenerators 65 verbunden. Der erste Schenkel 21 des zweiten Greifteils 20 ist mit einem zweiten Anschluß 67 des Generators 65 verbunden. Aufgrund der isolierenden Führung der ersten Schenkel 11 und 21 sind die Greifteile 10 und 20 bis zum Austreten aus der Führungshülse 30 vollständig voneinander isoliert. Im zusammengeklappten Zustand der Greifteile, wenn sich nichts zwischen den Greifteilen befindet, können sich die Greifflächen berühren, so daß ein elektrischer Kontakt zwischen den beiden Greifteilen 10 und 20 hergestellt ist. Zum Koagulieren wird mit den beiden Greifteilen Gewebe gegriffen, durch das dann ein entsprechender Koagulationsstrom von einem Greifteil über das Gewebe zum anderen Greifteil fließt.

Die Funktionsweise der Instrumentenspitze 2 wird anhand der folgenden Figuren noch genauer erläutert.

Fig. 2 zeigt eine vollständige Seitenansicht des chirurgischen Instrumentes 1 mit der Instrumentenspitze 2 und einem Handhabungsgriff 3.

In Fig. 3 ist ein Längsschnitt durch das in Fig. 2 dargestellte chirurgische Instrument 1 entlang der Linie III-III dargestellt. Anhand dieses Längsschnittes ist der Aufbau des Handhabungsgriffes 3 und insbesondere die Funktionsweise erkennbar.

Die von den Greifteilen 10 und 20 ausgehenden ersten Schenkel 11 bzw. 21 erstrecken sich durch das Rohr 40 hindurch bis in eine Betätigungseinrichtung 4, die in dem Handhabungsgriff 3 gelagert ist und zum Auseinander- bzw. Zusammenklappen der Greifteile 10 und 20 entgegen bzw. in Greifrichtung dient. Mittels einer Schraubenfeder 60 wird die Betätigungseinrichtung 4 aus dem Handhabungsgriff bis zu einem Anschlag gedrückt, wodurch auf die in der Betätigungseinrichtung 4 befestigten ersten Schenkel 11 und 21 der Greifteile 10 bzw. 20 eine Zugkraft in Längsrichtung des Rohres 40 und der Führungshülse 30 ausgeübt wird, so daß die Greifteile 10 und 20 zusammengeklappt sind und die Vorsprünge 33, 34 der äußeren Schenkel 12, 22 an der Hülse 30 anliegen. Dies entspricht sozusagen der Ruhestellung des Instrumentes.

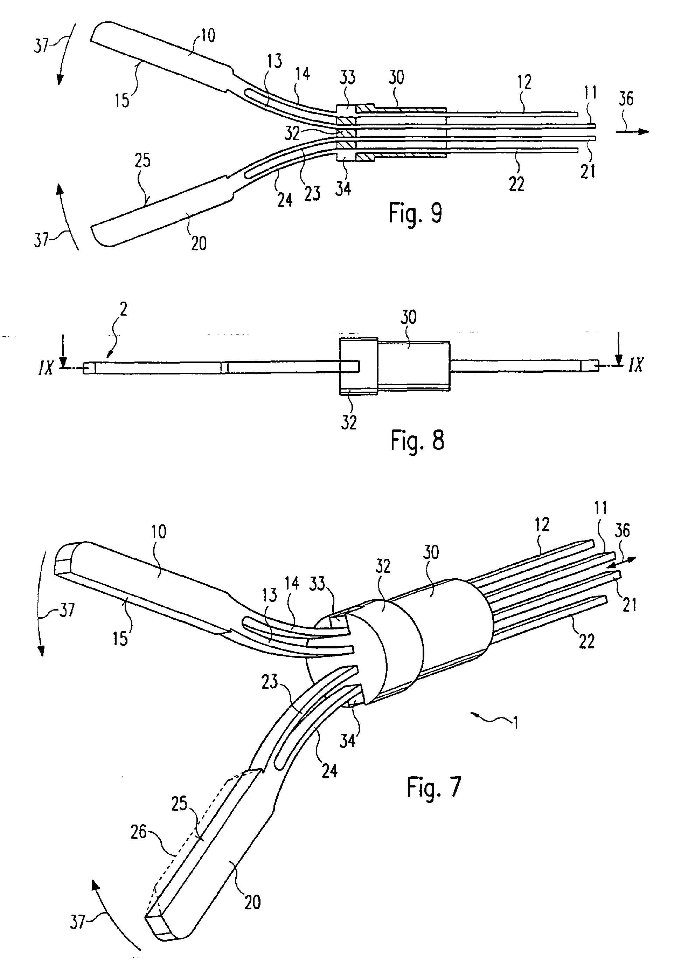

Durch Druck auf die Betätigungseinrichtung 4, genauer gesagt durch Hineindrücken der Betätigungseinrichtung 4 in den Handhabungsgriff 3 unter Zusammenpressen der Schraubenfeder 60, wird auf die ersten Schenkel 11 und 21 eine Schubkraft ausgeübt, durch welche die ersten Schenkel 11 und 21 in Längsrichtung des Rohres 40 und der Führungshülse 30 vom Handhabungsgriff 3 weg geschoben oder gedrückt werden. Dies bewirkt an der Instrumentenspitze 2 um die Befestigung der zweiten Schenkel 12 und 22 an der Führungshülse 30 ein Drehmoment, so daß die aus der Führungshülse 30 an der Instrumentenspitze 2 herausragenden Greifteile 10 und 20 an ihren Biegebereichen 13, 14 und 23, 24 nach außen gebogen werden, wodurch die Greifteile 10 und 20 entgegen der Greifrichtung auseinander klappen. Nun kann Gewebe von den beiden Greifteilen 10 und 20 umfaßt und durch Wegnahme des Drucks von der Betätigungseinrichtung 4 gegriffen werden. Ohne Handhabungsdruck drückt die Schraubenfeder 60 die Betätigungseinrichtung 4 aus dem Handhabungsgriff 3 wieder heraus, wodurch auf die ersten Schenkel 11 und 21 eine Zugkraft ausgeübt wird, welche die Greifteile 10 und 20 in Greifrichtung zusammen klappen und das Gewebe fassen bzw. greifen läßt. Das Gewebe ist damit zwischen den beiden Greifflächen der Greifteile eingeklemmt. Dann, wenn die Schenkel in Öffnungsrichtung der Greifteile vorgebogen sind, (s. Fig. 7) kann bei entsprechender Dimensionierung der Schraubenfeder die gesamte Anordnung auch ohne Verklebung nur durch die so auftretende Vorspannung zusammengehalten werden.

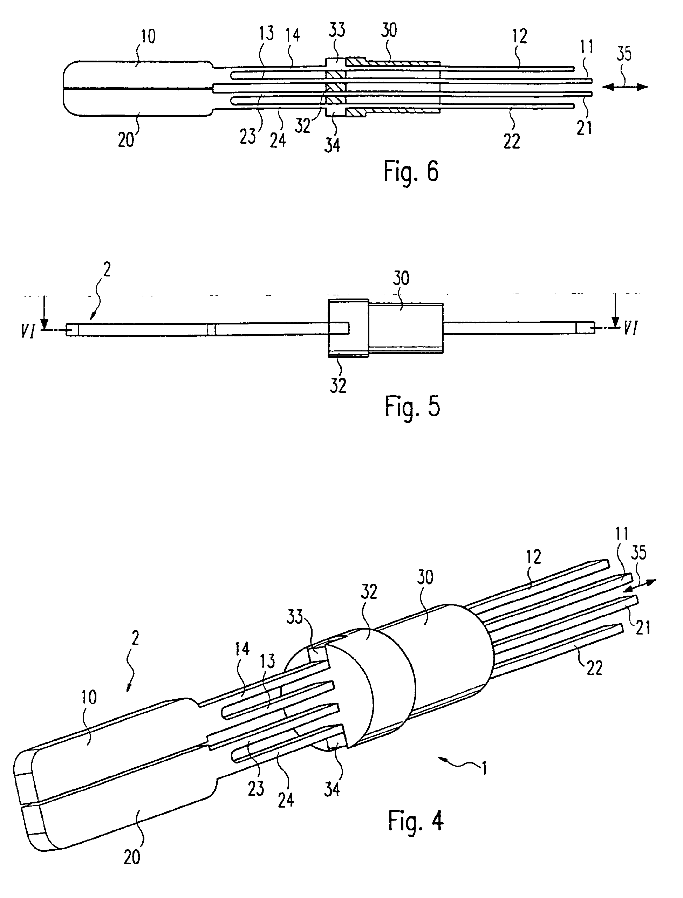

Fig. 4 zeigt ein zweites Ausführungsbeispiel des erfindungsgemäßen chirurgischen Instruments mit zusammengeklappten Greifteilen in perspektivischer Ansicht. Im Unterschied zum ersten Ausführungsbeispiel sind nur die zwei Greifteile 10 und 20, eine Führungshülse 30 und deren Kappenabschnitt 32 dargestellt. Zudem sind die Greifflächen der zwei Greifteile 10 und 20 glatt, d.h. unprofiliert.

Die ersten, innneren Schenkel 11 und 21 sowie die zweiten, äußeren Schenkel 12 und 22 der Greifteile 10 und 20 sind vollständig innerhalb der Führungshülse angeordnet, wodurch eine Verschmutzung oder Verunreinigung im wesentlichen verhindert und eine exakte Führung sichergestellt wird. Da es jedoch nicht ausgeschlossen werden kann, dass Schmutz wie Flüssigkeiten, Blut oder dergleichen Stoffe durch Kappilarwirkung in das Instrument gelangen, ist dieses mit einem Spülkanal zur Reinigung vorgesehen.

Die zweiten Schenkel 12 und 22 weisen Vorsprünge 33 bzw. 34 auf, die eine Bewegung der zweiten Schenkel 12 und 22 in Längsrichtung in die Führungshülse hinein verhindern. Die Vorsprünge 33 und 34 sind in dafür vorgesehenen Ausnehmungen des Kappenabschnittes 32 angeordnet, so daß außerdem ein Verdrehen der Greifteile 10 und 20 in der Führungshülse 30 verhindert wird. Ferner dienen die Vorsprünge zum Festmachen der zweiten Schenkel 12 und 22 an der Führungshülse 30. Hierzu können die Vorsprünge 33 und 34 beispielsweise an der Führungshülse 30 und/oder Abdeckkappe 32 auch angeklebt oder in die Aussparungen der Abdeckkappe 32 eingeklemmt sein.

Fig. 5 zeigt eine Seitenansicht des in Fig. 4 dargestellten zweiten Ausführungsbeispiels des chirurgischen Instrumentes.

Fig. 6 stellt einen Teilschnitt längs der Linie VI - VI des in Fig. 5 dargestellten Ausführungsbeispiels dar. Die Führungshülse 30 besteht aus einem isolierenden Material, insbesondere einem Kunststoff. Die ersten, inneren Schenkel 11 und 21 der beiden Greifteile 10 und 20 sind länger ausgebildet als die zweiten, äußeren Schenkel 12 und 22. Die Vorsprünge 33 und 34 der zweiten Schenkel 12 bzw. 22 der beiden Greifteile 10 bzw. 20 stoßen an die Führungshülse 30, so daß sie nicht in die Führungshülse hinein bewegbar sind.

Zum Auseinander- oder Zusammenklappen der Greifteile 10 und 20 werden die längeren ersten Schenkel 11 und 21 in der durch den Doppelpfeil 35 angedeuteten Bewegungsrichtung bewegt.

Fig. 7 stellt das in Fig. 4 dargestellte Ausführungsbeispiel des Instrumentes mit auseinander geklappten Greifteilen dar, wobei die Schenkel 13, 14, 23, 24 in Öffnungsrichtung der Greifteile 10, 20 vorgebogen sind. In Ruhestellung sind also die Greifteile des Instruments auseinandergeklappt. Zum Zusammenklappen der Greifteile werden die beiden inneren oder ersten Schenkel 11 und 21 in Richtung des Pfeiles 36 bewegt. Die Bewegung bewirkt ein Drehmoment um die festgemachten zweiten Schenkel 12 bzw. 22, wodurch sich die Greifteile 10 und 20 an den Biegebereichen 13 und 14 bzw. 23 und 24 der Schenkel 11 und 12 bzw. 21 und 22 biegen, so daß die Greifteile 10 und 20 in der Greifrichtung 37 zusammenklappen. Gut zu erkennen sind die Greifflächen 15 und 25 des ersten bzw. zweiten Greifteils 10 bzw. 20. Wenn zumindest eine der beiden Greifflächen 15 oder 25 als Schneide ausgebildet ist, kann das Instrument 1 auch zum Schneiden von Gewebe bzw. als Schere eingesetzt werden. In Fig. 7 ist die Ausbildung der Greiffläche 25 als Schneide 26 gestrichelt angedeutet.

Fig. 8 zeigt schließlich eine Seitenansicht des in Fig. 7 dargestellten Instrumentes und Fig. 9 einen Schnitt längs der Linie IX - IX des in Fig. 8 dargestellten Instrumentes.

### Bezugszeichenliste

- 1: chirurgisches Instrument
- 2: Instrumentenspitze
- 3: Handhabungsgriff
- 4: Betätigungseinrichtung
- 10: erstes Greifteil
- 11: erster Schenkel
- 12: zweiter Schenkel
- 13: Biegebereich
- 14: Biegebereich
- 15: Greiffläche
- 20: zweites Greifteil
- 21: erster Schenkel
- 22: zweiter Schenkel
- 23: Biegebereich
- 24: Biegebereich
- 25: Greiffläche
- 26: Schneide
- 30: Führungshülse
- 31: Abstandselement
- 32: Kappenabschnitt
- 33: Vorsprung
- 34: Vorsprung
- 35: Doppelpfeil
- 36: Bewegungsrichtung der ersten Schenkel
- 37: Greifrichtung
- 40: Rohr
- 50: Schlauch
- 51: Schlauch
- 60: Schraubenfeder
- 65: HF-Stromgenerator
- 66: erster Anschluß
- 67: zweiter Anschluß

## Patentansprüche

1. Chirurgisches Instrument (1), insbesondere Pinzette oder Zange für die minimalinvasive Chirurgie, umfassend:
zwei Greifteile (10, 20), die zum Greifen in und entgegen einer Greifrichtung (37) zusammen bzw. auseinander klappbar sind, wobei
die Greifteile (10, 20) an einem Ende einen ersten, inneren und einen zweiten, äußeren Schenkel (11, 12, 21, 22) mit jeweils einem Biegebereich (13, 14, 23, 24) aufweisen, die in der Greifrichtung voneinander beabstandet sind, wobei mindestens die ersten Schenkel (11, 21) der Greifteile (10, 20) innerhalb einer Führungshülse (30) angeordnet und in Längsrichtung der Führungshülse relativ zu den zweiten Schenkeln (12, 22) der Greifteile (10, 20) derart bewegbar sind, dass bei einer Relativbewegung der Schenkel zueinander in deren Längsrichtung das jeweilige Greifteil (10, 20) in Greifrichtung bewegbar ist,
**dadurch gekennzeichnet, dass**
die ersten Schenkel (11, 21) der Greifteile (10, 20) elektrisch voneinander isoliert sind, und dass
der erste Schenkel (11) des ersten Greifteiles (10) mit einem ersten Anschluss (66) und der erste Schenkel (21) des zweiten Greifteiles (12) mit einem zweiten Anschluss (67) eines HF-Stromgenerators (65) verbindbar sind.

2. Chirurgisches Instrument nach Anspruch 1,
**dadurch gekennzeichnet, dass**
der zweite Schenkel (12, 22) des Greifteils (10, 20) an der Führungshülse (30) festgemacht ist.

3. Chirurgisches Instrument nach Anspruch 1 oder 2,
**dadurch gekennzeichnet, dass**
jedes Greifteil (10, 20) mitsamt seinen Schenkeln (11, 12, 21, 22) einstückig aus einem leitfähigen, insbesondere metallenen Material hergestellt ist.

4. Chirurgisches Instrument nach einem der vorhergehenden Ansprüche,
**dadurch gekennzeichnet, dass**
der erste Schenkel (11, 21) jedes Greifteils (10, 20) länger als der zweite Schenkel (12, 22) ist.

5. Chirurgisches Instrument nach einem der vorhergehenden Ansprüche,
**dadurch gekennzeichnet, dass**
der zweite Schenkel (12, 22) jedes Greifteils (10, 20) insbesondere mit der Innenwand der Führungshülse (30) fest verbunden, insbesondere verklebt ist.

6. Chirurgisches Instrument nach einem der vorhergehenden Ansprüche,
**dadurch gekennzeichnet, dass**
die Führungshülse (30) teilweise in ein Ende eines Schlauches oder insbesondere metallenen Rohres (40) eingeführt ist.

7. Chirurgisches Instrument nach Anspruch 6,
**dadurch gekennzeichnet, dass**
der Schlauch oder das Rohr (40) an dem der Führungshülse (30) gegenüber liegenden Ende mit einem Handhabungsgriff (3) zum Handhaben des Instrumentes (1) versehen ist.

8. Chirurgisches Instrument nach Anspruch 7,
**dadurch gekennzeichnet, dass**
die ersten Schenkel (11, 21) der Greifteile (10, 20) mit einer Betätigungseinrichtung (4) verbunden sind, die derart in dem Handhabungsgriff (3) gelagert ist, dass durch Einführen der Betätigungseinrichtung (4) in den Handhabungsgriff die ersten Schenkel in Längsrichtung der Führungshülse (30) und des Schlauches oder Rohres (40) vom Handhabungsgriff (3) weg bewegt werden, wodurch die Greifteile (10, 20) auseinander klappen und umgekehrt durch Herausziehen der Betätigungseinrichtung (4) aus dem Handhabungsgriff (3) die ersten Schenkel der Greifteile in Längsrichtung der Führungshülse und des Rohres zu der Betätigungseinrichtung (4) gezogen werden, wodurch die Greifteile zusammenklappen.

9. Chirurgisches Instrument nach Anspruch 8,
**dadurch gekennzeichnet, dass**
zwischen der Betätigungseinrichtung (4) und dem Handhabungsgriff (3) eine Spanneinrichtung (60) angeordnet ist, so dass die Betätigungseinrichtung ohne äußere Krafteinwirkung aus dem Handhabungsgriff gedrückt wird und die Greifteile zusammengeklappt sind.

10. Chirurgisches Instrument nach Anspruch 2,
**dadurch gekennzeichnet, dass**
der erste Schenkel (11, 21) durch eine Spanneinrichtung (60) in die Führungshülse (30) hineingezogen wird und ein Vorsprung (33, 34) am zweiten Schenkel (12, 22) vorgesehen ist, der ein Hineingleiten des Greifteils (10, 20) in die Führungshülse (30) verhindert.

## Claims

1. Surgical instrument (1), in particular pincers or forceps for minimally invasive surgery, comprising:
two gripping elements (10, 20), which for the purpose of gripping can be moved towards one another in a gripping direction (37) or apart from one another in an opposite direction, wherein
the gripping elements (10, 20) have at one end a first, inner, and a second, outer, limb (11, 12, 21, 22) each with a bending region (13, 14, 23, 24), which regions are spaced apart in relation to each other in the gripping direction, such that at least the first limb (11, 21) of the gripping element (10, 20) is disposed within a guide sleeve (30) and can be moved in the longitudinal direction of the guide sleeve relative to the second limb (12, 22) of the gripping element (10, 20) in such a way that during the relative movement of the limbs towards each other in their longitudinal direction, the associated gripping element (10, 20) can be moved in the gripping direction,
**characterised in that**
the first limbs (11, 21) of the gripping elements (10, 20) are electrically insulated from each other, and **in that**
the first limb (11) of the first gripping element (10) can be connected to a first connector (66) and the first limb (21) of the second gripping element (12) can be connected to a second connector (67) of an HF current generator (65).

2. Surgical instrument according to Claim 1,
**characterised in that**
the second limb (12, 22) of the gripping element (10, 20) is fixed to the guide sleeve (30).

3. Surgical instrument according to Claim 1 or 2,
**characterised in that**
each gripping element (10, 20) together with its limbs (11, 12, 21, 22) is manufactured in one piece from a conductive, in particular metallic, material.

4. Surgical instrument according to one of the previous claims,
**characterised in that**
the first limb (11, 21) of each gripping element (10, 20) is longer than the second limb (12, 22).

5. Surgical instrument according to one of the previous claims,
**characterised in that**
the second limb (12, 22) of each gripping element (10, 20) is fixedly connected, in particular is stuck, to the inner wall of the guide sleeve (30) in particular.

6. Surgical instrument according to one of the previous claims,
**characterised in that**
the guide sleeve (30) is partly inserted in one end of a hose or, in particular, a metal tube (40).

7. Surgical instrument according to Claim 6,
**characterised in that**
the hose or the tube (40) is provided on the end facing the guide sleeve (30) with a handle (3) for manipulating the instrument (1).

8. Surgical instrument according to Claim 7,
**characterised in that**
the first limbs (11, 21) of the gripping elements (10, 20) are connected to an actuating device (4), which is mounted in the handle (3) in such a way that, when the actuating device (4) is inserted into the handle, the first limbs are moved in a longitudinal direction of the guide sleeve (30) and of the hose or tube (40) away from the handle (3), causing the gripping elements (10, 20) to open, and, conversely, when the actuating device (4) is withdrawn from the handle (3), the first limbs of the gripping elements are pulled in a longitudinal direction of the guide sleeve and of the tube towards the actuating device (4), causing the gripping elements to close.

9. Surgical instrument according to Claim 8,
**characterised in that**
a tensioning device (60) is arranged between the actuating device (4) and the handle (3) so that the actuating device is pressed against the handle without any external effect of force and the gripping elements are closed.

10. Surgical instrument according to Claim 2,
**characterised in that**
the first limb (11, 21) is pulled into the guide sleeve (30) by a tensioning device (60) and a projection (33, 34) is provided on the second limb (12, 22), preventing the gripping element (10, 20) from sliding into the guide sleeve (30).

## Revendications

1. Instrument chirurgical (1), en particulier pincette ou pince pour la chirurgie minimalement invasive, comprenant deux parties de préhension (10, 20) qui peuvent être repliées ou déployées pour saisir dans et contre une direction de préhension (37), lesquelles parties de préhension (10, 20) présentent à une extrémité une première branche interne et une deuxième branche externe (11, 12, 21, 22) possédant chacune une zone de flexion (13, 14, 23, 24) espacées les unes des autres dans la direction de préhension, au moins les premières branches (11,21) des parties de préhension (10, 20) étant disposées dans une douille de guidage (30) et mobiles dans le sens longitudinal de la douille de guidage par rapport aux deuxièmes branches (12, 22) des parties de préhension (10, 20) de telle façon que, lors d'un mouvement relatif des branches l'une en direction de l'autre dans leur sens longitudinal, chaque partie de préhension (10, 20) peut se déplacer dans la direction de préhension, **caractérisé en ce que** les premières branches (11, 21) des parties de préhension (10, 20) sont électriquement isolées l'une de l'autre et **en ce que** la première branche (11) de la première partie de préhension (10) peut être reliée à une première connexion (66) et la première branche (21) de la deuxième partie de préhension (12) à une deuxième connexion (67) d'un générateur de courant haute fréquence (65).

2. Instrument chirurgical selon la revendication 1, **caractérisé en ce que** la deuxième branche (12, 22) de la partie de préhension (10, 20) est fixée sur la douille de guidage (30).

3. Instrument chirurgical selon la revendication 1 ou la revendication 2, **caractérisé en ce que** chaque partie de préhension (10, 20) est réalisée d'une seule pièce avec ses branches (11, 12, 21, 22) dans un matériau conducteur, en particulier métallique.

4. Instrument chirurgical selon l'une des revendications précédentes, **caractérisé en ce que** la première branche (11, 21) de chaque partie de préhension (10, 20) est plus longue que la deuxième branche (12, 22).

5. Instrument chirurgical selon l'une des revendications précédentes, **caractérisé en ce que** la deuxième branche (12, 22) de chaque partie de préhension (10, 20) est reliée fixement en particulier à la paroi intérieure de la douille de guidage (30), en particulier collée ;

6. Instrument chirurgical selon l'une des revendications précédentes, **caractérisé en ce que** la douille de guidage (30) est introduite en partie dans une extrémité d'une gaine ou en particulier d'un tube métallique (40).

7. Instrument chirurgical selon la revendication 6, **caractérisé en ce que** la gaine ou le tube (40) est muni à son extrémité opposée à la douille de guidage (30) d'une poignée de manipulation (3) servant à manipuler l'instrument (1).

8. Instrument chirurgical selon la revendication 7, **caractérisé en ce que** les premières branches (11, 21) des parties de préhension (10, 20) sont reliées à un dispositif actionneur (4) qui est supporté dans la poignée de manipulation (3) de telle façon que l'introduction du dispositif actionneur (4) dans la poignée de manipulation éloigne les premières branches de la poignée de manipulation (3) et de la gaine ou du tube (40) dans le sens longitudinal de la douille de guidage (30), ce qui a pour effet de déployer les parties de préhension (10, 20), et inversement, le retrait du dispositif actionneur (4) hors de la poignée de manipulation (3) tire les premières branches des parties de préhension dans le sens longitudinal de la douille de guidage et du tube en direction du dispositif actionneur (4), ce qui a pour effet de replier les parties de préhension.

9. Instrument chirurgical selon la revendication 8, **caractérisé en ce qu'**un dispositif tendeur (60) est disposé entre le dispositif actionneur (4) et la poignée de manipulation (3) de façon à forcer le dispositif actionneur à sortir de la poignée de manipulation sans appliquer de force extérieure et à replier les parties de préhension.

10. Instrument chirurgical selon la revendication 2, **caractérisé en ce que** la première branche (11, 21) est tirée par un dispositif tendeur (60) dans la douille de guidage (30) et **en ce qu'**il est prévu sur la deuxième branche (12, 22) une saillie (33, 34) qui empêche la partie de préhension (10, 20) de glisser à l'intérieur de la douille de guidage (30).
